# EUROPÄISCHE PATENTSCHRIFT

(11) **EP 0 750 459 B1**
(45) Veröffentlichungstag und Bekanntmachung des Hinweises auf die Patenterteilung: **03.03.1999**
(21) Anmeldenummer: 95924861.8
(22) Anmeldetag: 14.07.1995
(51) Int. Cl.: A01N 63/04, C12N 1/14

(54) **PILZISOLAT, PRÄPARAT ZUR BEKÄMPFUNG PFLANZENPATHOGENER PILZE, VERFAHREN ZU SEINER HERSTELLUNG SOWIE SEINE VERWENDUNG**
FUNGUS ISOLATE, PREPARATION FOR COMBATTING PLANT-PATHOGENIC FUNGI, PROCESS FOR PRODUCING IT AND ITS USE
ISOLAT DE CHAMPIGNON, PREPARATION POUR LUTTER CONTRE LES CHAMPIGNONS PATHOGENES DES PLANTES, SES PROCEDES DE PREPARATION ET SON UTILISATION

(30) Priorität: 14.01.1995 DE 19502065
(43) Veröffentlichungstag der Anmeldung: 02.01.1997
(73) Patentinhaber: PROPHYTA BIOLOGISCHER PFLANZENSCHUTZ GMBH, 23999 Malchow (DE)
(72) Erfinder: LÜTH, Peter, D-23970 Wismar (DE); EIBEN, Ute, D-23999 Malchow (DE)
(74) Vertreter: Baumbach, Friedrich, Dr.
(86) Internationale Anmeldenummer: DE9500926
(87) Internationale Veröffentlichungsnummer: WO9621358

(56) Entgegenhaltungen:
- EP-A- 0 466 133
- MYCOLOGY RESEARCH, Band 97, Nr. 10, Oktober 1993; C. SANDYS-WINSCH et al., Seiten 1175-1178
- PESTIC. SCIENCE, Band 37, 1993; J.M. WHIPPS et al., Seiten 309-313
- PLANT PATHOLOGY, Band 40, 1991; S.P. BUDGE et al., Seiten 59-66
- PROCEEDINGS OF NATO ADV, RESEARCH WORKSHOP ON BIOLOGICAL CONTROL OF PLANT DISEASES, E.C. TJAMOS et al. (eds.), 1992, New York, NY (US); P. DAVET et al., Seiten 427-430

## Beschreibung

Gegenstand der Erfindung ist das Isolat CON/M/91-08 der Pilzart *Coniothyrium minitans* sowie dessen Anwendung zur Bekämpfung sklerotienbildender Pflanzenkrankheitserreger, insbesondere *Sclerotinia sclerotiorum*.

Die durch den Krankheitserreger *S*. *sclerotiorum* verursachte Fäule an den verschiedensten Pflanzenorganen tritt an vielen Kulturpflanzen, wie z.B. Sonnenblume, Tomate, Tabak, Salat und Raps, auf. Sie verursacht z.T. sehr hohe Ertrags- und Lagerverluste.
Eine chemische Bekämpfung der Krankheit ist in den meisten Fällen unbefriedigend oder überhaupt nicht möglich, da der Krankheitserreger in Form seiner Dauerkörper, den Sklerotien, im Boden überdauert, von wo aus er seine Wirtspflanzen mit Hilfe verschiedener möglicher Infektionsmodi befällt.

So keimt der Pilz einerseits mit seinen Fruchtkörpern, den Apothezien, aus den Sklerotien aus und bildet Sporen, die die oberirdischen Pflanzenteile infizieren. Andererseits kann Pilzmyzel aus den Sklerotien auswachsen, das in der Lage ist sowohl ober- als auch unterirdische Pflanzenteile zu infizieren.

Während im ersten Falle eine chemische Bekämpfung in bestimmten Fällen (z.B. beim Winterraps) eingeschränkt möglich ist, kann eine Infektion über das Myzel bisher chemisch nicht bekämpft werden.

Durch den Einsatz mikrobieller Antagonisten, zu denen auch *Coniothyrium minitans* gehört, die die Zerstörung der Sklerotien im Boden bewirken sollen, könnte einerseits auf eine chemische Bekämpfung, wo sie heute noch angewandt wird, verzichtet werden, andererseits wäre in vielen Anwendungsfällen eine Bekämpfung von *S*. *sclerotiorum* überhaupt erst möglich.

*Coniothyrium minitans* ist ein im Boden natürlich vorkommender Pilz mit weltweiter Verbreitung. Es gibt zahlreiche Berichte über die Isolierung dieses Pilzes aus Sklerotien von *S*. *sclerotiorum* und *S*. *trifoliorum*. Der Nachweis, daß *C*. *minitans* die Sklerotien der Pilzgattung *Sclerotinia* zu schädigen vermag, ist in vielen Labor- und Felduntersuchungen erbracht worden. Es gibt auch Untersuchungen (Trutmann, P. et al, Soil Biology and Biochemistry 12, 461-465 (1980), Tiedemann, A. von et al, Mitteilungen aus der Biologischen Bundesanstalt für Land- und Forstwirtschaft, Berlin-Dahlem, Heft 301, 361 (1994) und Budge, S.P. et al, Plant Pathologie 40, 59-66 (1991)), mit denen eine Reduzierung der Sklerotien-Verseuchung des Boden nach einer Applikation von *C*. *minitans* nachgewiesen wurde.

Ein Nachteil der Verwendung von *Coniothyrium minitans* besteht jedoch darin, daß zum gegenwärtigen Zeitpunkt eine kontrollierte und gezielte Anwendung nicht möglich ist.

Der Erfindung liegt die Aufgabe zugrunde, ein Isolat der Pilzart *Coniothyrium minitans* zu finden, das besonders gut zur Bekämpfung von pflanzenpathogenen Pilzen geeignet ist und eine kontrollierte und gezielte Anwendung ermöglicht.

Ein solches Isolat wurde mit dem Isolat CON/M/91-08 gefunden und unter der Nummer DSM 9660 bei der Deutschen Sammlung von Mikroorganismen und Zellkulturen, Braunschweig, Bundesrepublik Deutschland, hinterlegt.

Das erfindungsgemäße Isolat wurde als Einsporlinie aus 32 Einsporlinien selektiert und hat eine besonders hohe antagonistische Aktivität gegen *S*. *sclerotiorum* sowie eine sehr gute Befähigung zur Pyknidienbildung. CON/M/91-08 wächst in vitro auf unterschiedlichen Nährböden. Das Isolat ist in Abhängigkeit vom verwendeten Nährboden sowie einer Wachstumstemperatur von 22 °C und Kultur unter Lichtabschluß wie folgt charakterisiert:

| **Kultureigenschaften** | **Kartoffel- Dextrose- Agar** | **Biomalz-Agar** | **Biomalz-Agar + Hefe (3 g/l)** | **Czapek-Dox- Agar** |
|---|---|---|---|---|
| **Kulturfarbe** | oberseitig: | oberseitig: | oberseitig: | oberseitig: |
| | hellgrau | dunkeloliv mit weißem bis hyalinem Rand | schmutzig grau , zum Zentrum hin oliv | schmutzig weiß |
| | unterseitig: | | | |
| | schmutzig weiß, mit dunkelbraunen Flecken | | | unterseitig: |
| | | unterseitig: | | dunkeloliv mit weißem Rand |
| | | wie oberseitig | unterseitig: | |
| | | | dunkeloliv mit weißem Rand | |
| **Form des Myzels** | flach aufliegendes spärlich entwickeltes Myzel | flach aufliegendes spärlich entwickeltes Myzel | Myzelschicht 1,0 - 1,5 mm dick, wattig | Myzelschicht bis 1,0 mm dick, flockig |
| **Myzelwachstum (n = 10)** | 4,6 cm/10 Tage | 4,3 cm/10 Tage | 5,0 cm/10 Tage | 2,2 cm/10 Tage |
| **Pyknidienbildung (n = 50)** | 44 Pyknidien/cm² | 82 Pyknidien/cm² | 67 Pyknidien/cm² | 23 Pyknidien/cm² |
| **Pyknidiendurchmesser (n = 50)** | 192 µm | 270 µm | 370 µm | 205 µm |
| **Konidiegröße (n = 100)** | 5,27 x 3,51 µm | 6,15 x 4,35 µm | 5,68 x 3,89 µm | 5,98 x 3,72 µm |

Zum Nachweis des Isolates CON/M/91-08 wurde außerdem eine RAPD-Analyse nach Williams et al., 1990, Nucleic Acid Res. 18, 6531 durchgeführt. Als RAPD-Primer dienten DAF4, DAF6, DAF8, DAF9.

Das erfindungsgemäß einzusetzende Pilzisolat kann auf geeigneten Substraten, wie Getreidekörnern, Getreidekleie, Stroh oder anderen Pflanzenmaterialien, oder auch mit Hilfe von in der Mykologie üblichen Agar-Nährböden, wie Kartoffel-Dextrose-Agar oder Malz-Pepton-Agar, oder auf geeigneten Trägermaterialien mit einem Nährsubstratzusatz sowie in flüssigen Nährsubstraten ohne Agarzusatz kultiviert werden.

Gegenstand der vorliegenden Erfindung sind auch Pflanzenschutzmittel, die das Isolat CON/M/91-08 in Form seiner Pyknidiosporen, seines Myzels oder sonstiger Pilzbestandteile neben üblichen Zusatzstoffen, welche der Suspendierung oder Formulierung dienen, enthalten.

Die erfindungsgemäßen Mittel können als versprühbare Lösungen (Dispersionen), emulgierbare Konzentrate, Spritzpulver, Stäubemittel, Beizmittel, Dispersionen, Granulate oder Mikrogranulate in den üblichen Zubereitungen angewandt werden.

Die handelsüblich als Konzentrat vorliegenden Mittel werden zur Anwendung im allgemeinen verdünnt. Dieses geschieht bei wasserdispergierbaren Granulaten, Spritzpulvern, emulgierbaren Konzentraten sowie bei Dispersionen z.B. mit Wasser. Andere Formulierungen, wie Stäube, Beizmittelzubereitungen oder Granulate, werden vor ihrer Anwendung gebräuchlicher Weise nicht verdünnt. Das Mittel kann zum Zwecke seiner Anwendung unter bestimmten Umständen auch mit anderen, auch chemischen, Pflanzenschutzmitteln, wie Fungiziden, Insektiziden, Herbiziden oder Akariziden, oder auch mit Wachstumsregulatoren oder Düngemitteln gemischt werden.

Anhand der folgenden Beispiele soll die Erfindung im Hinblick auf einige wichtige Eigenschaften näher charakterisiert werden.

### Beispiel 1

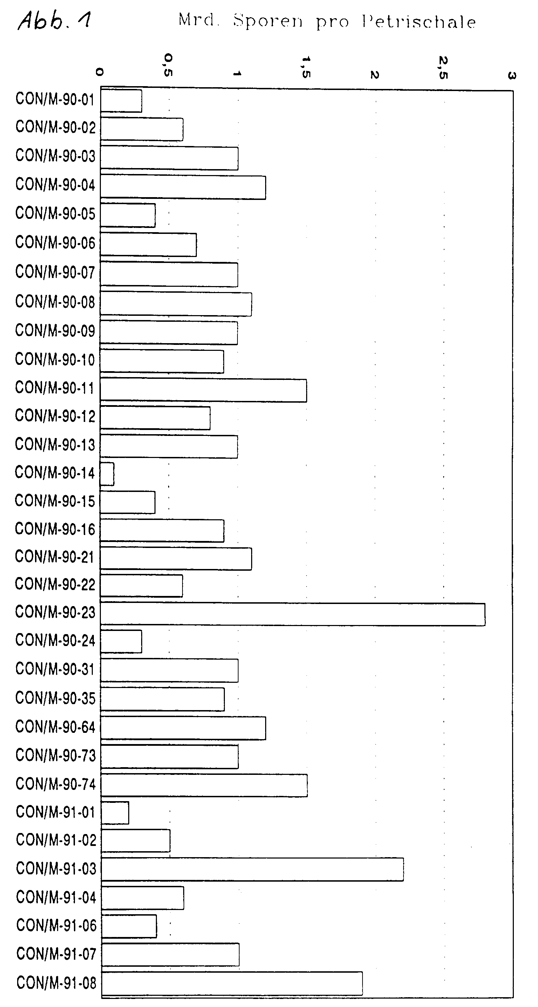

Aus Sklerotien der Pilzarten *S*. *Sclerotiorum* und *S*. *trifoliorum* von verschiedenen Standorten wurden zahlreiche Isolate des Pilzes *C*. *minitans* gewonnen, indem die Sklerotien oberflächlich sterilisiert und auf einen Agarnährboden aufgelegt wurden. Die mit ihrem Myzel auswachsenden Pilze wurden bestimmt und alle zur Art *C*. *minitans* gehörenden Isolate in eine Isolatesammlung übernommen. Mit diesen Isolate wurde eine Selektion hinsichtlich ihrer Befähigung zur Sporenbildung durchgeführt. Diese erfolgte in 2 Selektionsschritten. Dazu wurden von den ursprünglich gewonnenen 23 Isolaten Suspensionen aus Pyknidiosporen hergestellt und auf einem Biomalz-Agar mit Zusatz von Hefe-Extrakt ausgestrichen. Nach beginnender Pyknidienbildung auf den verwendeten Agarplatten zeigten sich einige Kolonien mit besonders vielen gut ausgebildeten Pyknidien. Aus diesen wurde je eine Pyknidie gewonnen und als Linie weitergeführt. Insgesamt konnten 528 Linien gewonnen werden, die in einer Folgekultur hinsichtlich ihrer Pyknidienbildungsrate untersucht und selektiert wurden. Dabei wurden 95 Linien gewonnen und in einem 2. Selektionsschritt eingesetzt. Bei der 2. Selektion wurden aus Kolonien mit einer besonders starken Pyknidienbildung Einsporlinien gewonnen und in einer Folgekultur selektiert. Als Ergebnis standen 32 Einsporlinien mit einer sehr guten Befähigung zur Pyknidienbildung zur Verfügung. Die Befähigung dieser Linien zur Bildung von Pyknidiosporen in Agar-Kultur ist in Abb.1 dargestellt.

### Beispiel 2

32 Einsporlinien von *C*. *minitans* mit einer besonders guten Befähigung zur Pyknidienbildung (Beschreibung unter Beispiel 1) waren Ausgangspunkt für die Selektion einer Linie, die sowohl eine gut Befähigung zur Pyknidienbildung als auch eine gut antagonistische Wirkung in sich vereinigt. Zur Selektion dieser Linie wurden Versuchsgefäße mit Quarzsand befüllt und mit einer Pyknidiosporensuspension (Konzentration 1x 10⁴ Sporen/ml) befeuchtet (erdfeucht). Im Anschluß daran wurden je 25 natürlich gebildete Sklerotien vom Winterraps in 4 Wiederholungen 1 cm tief eingebettet und bei folgenden Temperaturen im Klimaschrank inkubiert:
4 Wochen - 15 °C
8 Wochen - 12 °C

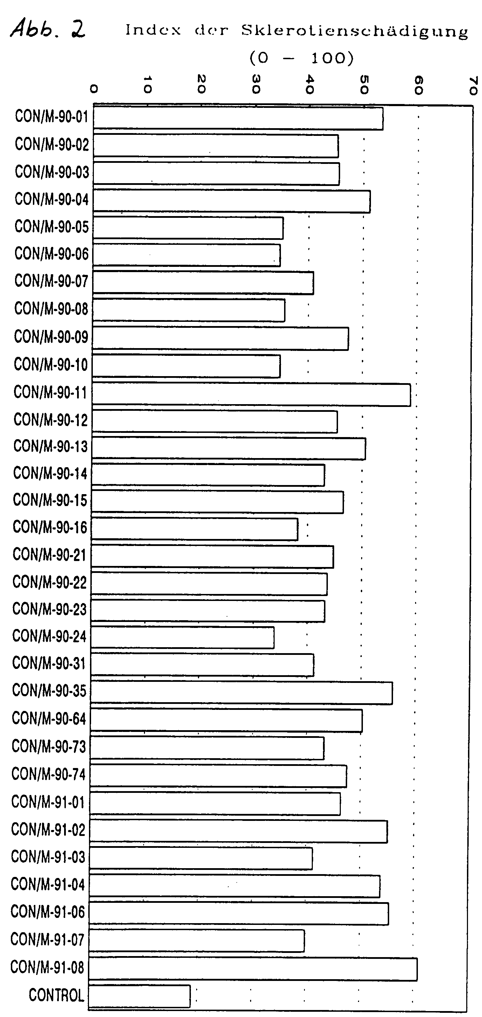

Damit wurden die im Boden herrschenden Temperaturbedingungen des Zeitraumes von Mitte August bis Mitte November simuliert. Die Wirkungsbedingungen für die zu bewertenden *Coniothyrium*-Isolate wurden mit Absicht ungünstig gestaltet, um solche Isolate selektieren zu können, die auch bei ungünstigen äußeren Einflüssen eine gute Wirkung aufweisen. Die Bewertung der antagonistischen Befähigung der Einsporlinien erfolgte anhand der Zerstörung der Sklerotien nach Ablauf der Inkubation (Abb.2).

Die Einsporlinie CON/M/91-08 verursachte mit einem Index von 60,8 die stärkste Schädigung der Sklerotien. Über 90 % der Sklerotien war geschädigt, die meisten davon stark.

### Beispiel 3

Nach Ausbringung von 300 Sklerotien pro m² sowie ihrer flachen Einarbeitung mittels Ackerfräse in den Erdboden wurden Suspensionen von Pyknidiosporen in folgenden Varianten auf die Bodenoberfläche gesprüht und ebenfalls eingearbeitet.

| | |
|---|---|
| 1. CON/M/91-08 | Konz.: 1 x 10⁶ |
| 2. CON/M/91-08 | Konz.: 5 x 10⁶ |
| 3. CON/M/91-08 | Konz.: 1 x 10⁷ |
| 4. Isolat VIII 2 | Konz.: 5 x 10⁶ |

Bei dem Isolat VIII 2 handelte es sich um eines der ursprünglich gewonnenen Isolate, das keiner weiteren Selektion unterworfen war. Es wurden jeweils 50 ml Suspension/m² ausgebracht. Zusätzlich zu den Varianten wurde eine mit *Sclerotinia sclerotiorum* verseuchte aber nicht mit Antagonisten behandelte Variante als Kontrolle mitgeführt. Die Prüfung wurde als Blockanlage mit 12 m² großen Parzellen in 4facher Wiederholung angelegt. zwischen den Parzellen wurden Distanzstreifen angelegt, um die Verbreitung der Ascosporen von *S*. *sclerotiorum* mit dem Wind über die Parzellengrenzen hinaus möglichst gering zu halten.
Die Auswertung erfolgte im Juli/August des darauffolgenden Jahres anhand des Befalls der Parzellen (Anteil befallener Pflanzen in %) sowie des Parzellenertrages. Die Ergebnisse sind in folgender Tabelle dargestellt.

**Tabelle 1**

| **Variante** | **befallene Pflanzen** | **Parzellenertrag** | |
|---|---|---|---|
| | | **kg/Parz.** | **% zur Kontr.** |
| CON/M/91-08 (1 x 10⁶) | 20,3 % | 5,89 | 106,3 |
| CON/M/91-08 (5 x 10⁶) | 7,4 % | 6,65 | 120,0 |
| CON/M/91-08 (1 x 10⁷) | 5,3 % | 6,77 | 122,2 |
| Isolat VIII 2 (5 x 10⁶) | 18,8 % | 5,91 | 106,7 |
| Kontrolle | 32,5 % | 5,54 | 100,0 |

Durch die Einarbeitung von 50 ml des Isolates CON/M/91-08 in die oberste Bodenschicht konnte bereits bei Anwendung einer Pyknidiosporenkonzentration von 5 x 10⁶ Sporen/ml eine deutliche Reduzierung des Sclerotinia-Befalls festgestellt werden. Diese hatte signifikante Erhöhung des Parzellenertrages zur Folge.

### Beispiel 4

RAPD-Analyse:
Williams et al., 1990, Nucleic Acid Res. 18, 6531

Es wurden durch TÜV Energie und Umwelt, Fachgruppe Biologische Sicherheit mittels RAPD-Analyse 6 Isolate des *Coniothyrium minitans* Pilzes untersucht,
Isolat 10, Isolat 11, Isolat 20, Isolat 21 (CON/M/91-08), Isolat 22, Isolat 32 mit dem Ziel der Unterscheidung des Isolates 21 von allen anderen.

Ergebnis: Im Vortest wurden 8 RAPD-Primer auf Differenzierungsfähigkeit zwischen Isolat 21 und 22 geprüft. Drei der Primer differenzierten nicht. Für die Analyse aller Isolate wurden anschließend die Primer DAF4, DAF6, DAF8 und DAF9 eingesetzt.

Die mit DAF4 und DAF8 erzeugten Muster differenzierten Isolat 21 eindeutig von allen anderen. DAF6 und DAF9 differenzierten ebenfalls zwischen Isolat 21 und den anderen Isolaten. Letztere ergeben aber mit diesen Primern unterschiedliche Muster, die z.T. dem Muster von Isolat 21 ähnlich sind.

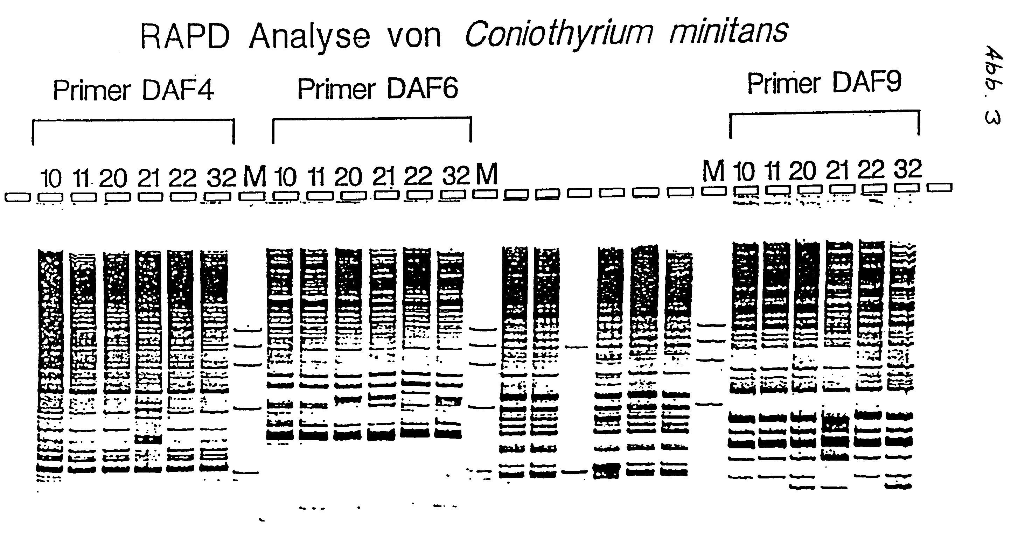

Abb. 3 - Elektrophorese-Gele

## Patentansprüche

1. Das Isolat CON/M/91-08 - DSM 9660 der Pilzart *Coniothyrium minitans* Campbell einschließlich jeder von diesem Isolat abstammenden Mutante.

2. Präparat zur Bekämpfung von pflanzenpathogenen Pilzen, dadurch gekennzeichnet, daß eine fungizid wirksame Menge des Isolates CON/M/91-08 nach Anspruch 1 darin enthalten ist.

3. Präparat nach Anspruch 2, dadurch gekennzeichnet, daß die fungizid wirksame Menge des Isolates CON/M/91-08 aus Konidien, Chlamydosporen, Fragmenten von Hyphen oder aus einer Mischung dieser Bestandteile besteht.

4. Präparat nach Anspruch 2 und 3, dadurch gekennzeichnet, daß das Isolat CON/M/91-08 mit an sich üblichen Hilfs- und Trägerstoffen formuliert ist.

5. Verfahren zur Bekämpfung von pflanzenpathogenen Pilzen, dadurch gekennzeichnet, daß ein Präparat nach Anspruch 2 bis 4 auf den zu bekämpfenden Pilz oder Pilzorgane oder auf die vor einem Pilzbefall zu schützenden Pflanzen oder Pflanzenteile einschließlich Saatgut oder auf bzw. in den Boden gebracht wird.

6. Verfahren nach Anspruch 5, dadurch gekennzeichnet, daß das Isolat CON/M/91-08 als Konidiensuspension im Sprühverfahren angewandt wird.

7. Verwendung des Präparates nach Anspruch 2 bis 4, zur Bekämpfung sklerotienbildender Pilze.

8. Verwendung nach Anspruch 7, dadurch gekennzeichnet, daß die Pilzart *Sclerotinia sclerotiorum* bekämpft wird.

## Claims

1. Isolate CON/M/91-08 - DSM 9660 of the fungal species *Comothyrium minitans Campbell* including each of the mutants descending from this isolate

2. Preparation for combatting phytopathogenic fungi wherein a fungicidally effective quantity of isolate CON/M/91-08 according to claim 1 is contained

3. Preparation according to claim 2 wherein a fungicidally effective quantity of isolate CON/M/91-08 consists of conidia, chlamydial spores, fragments of hyphas or of a mixture of these components

4. Preparation according to claims 2 and 3 wherein isolate CON/M/91-08 is formulated of, in principle, usual auxiliary agents and carriers

5. Procedure for combatting phytopathogenic fungi wherein a preparation according to claims 2 to 4 is placed on the fungus or the fungal organs to be combatted or on the plants or parts of plants including seeds to be protected against a fungal infection or on and in the soil respectively

6. Procedure according to claim 5 wherein isolate CON/M/91-08 is applied by spraying as conidia suspension

7. Use of the preparation according to claims 2 to 4 for combatting sclerotia forming fungi

8. Use according to claim 7 wherein the fungal species *sclerotinia sclerotiorum* is combatted

## Revendications

1. Isolat CON/M/91-08-DSM 9660 de l'espèce de champignon *Coniothyrium minitans* Campbell, y compris tous les mutants descendant de cet isolat.

2. Préparation pour la lutte contre les champignons phytopathogènes, caractérisée en ce quelle contient une quantité de l'isolat CON/M/91-08 selon la revendication 1 suffisante pour exercer une action fongicide.

3. Préparation selon la revendication 2, caractérisée en ce que la quantité de l'isolat CON/M/91-08 ayant une action fongicide se compose de conidies, de chlamydospores, de fragments d'hyphes ou d'un mélange de ces organes.

4. Préparation selon les revendications 2 et 3, caractérisée en ce que l'isolat CON/M/91-08 est associé à des adjuvants et des véhicules courants.

5. Procédé de lutte contre les champignons phytopathogènes, caractérisé en ce qu'une préparation selon les revendications 2 à 4 est appliquée sur le champignon ou des organes du champignon à combattre ou sur les plantes ou des parties des plantes à protéger contre une infection fongique, y compris leurs semences, ou encore sur ou dans le sol.

6. Procédé selon la revendication 5, caractérisé en ce que l'isolat CON/M/91-08 est utilisé en pulvérisations sous la forme d'une suspension de conidies.

7. Utilisation du procédé selon les revendications 2 à 4 pour la lutte contre les champignons provoquant des sclérotinioses.

8. Utilisation selon la revendication 7, caractérisée en ce que l'espèce visée est le champignon *Sclerotinia sclerotiorum*.
